# EUROPÄISCHE PATENTSCHRIFT

(11) **EP 2 994 152 B1**
(45) Veröffentlichungstag und Bekanntmachung des Hinweises auf die Patenterteilung: **15.08.2018**
(21) Anmeldenummer: 14718515.1
(22) Anmeldetag: 16.04.2014
(51) Int. Cl.: A61K 51/08, C07K 7/08

(54) **KONJUGATE ZUM SCHUTZ VOR NEPHROTOXISCHEN WIRKSTOFFEN**
CONJUGATES FOR PROTECTION FROM NEPHROTOXIC ACTIVE SUBSTANCES
CONJUGUÉS OFFRANT UNE PROTECTION CONTRE LES SUBSTANCES ACTIVES NÉPHROTOXIQUES

(30) Priorität: 07.05.2013 EP 13002431
(43) Veröffentlichungstag der Anmeldung: 16.03.2016
(73) Patentinhaber: Merck Patent GmbH, 64293 Darmstadt (DE)
(72) Erfinder: KUEBELBECK, Armin, 64625 Bensheim (DE); LARBIG, Gregor, 63571 Gelnhausen (DE); ARNOLD, Stefan, 64839 Muenster (DE); MIER, Walter, 64673 Bensheim (DE); HABERKORN, Uwe, 67823 Schwetzingen (DE)
(86) Internationale Anmeldenummer: PCT/EP2014/001025
(87) Internationale Veröffentlichungsnummer: WO 2014/180533

(56) Entgegenhaltungen:
- WO-A1-2007/022774
- WO-A1-2011/009539
- NORIKO IHARA ET AL: "Amplification of Inhibitory Activity of Catechin against Disease-Related Enzymes by Conjugation on Poly([epsilon]-lysine)", BIOMACROMOLECULES, Bd. 5, Nr. 5, 1. September 2004 (2004-09-01), Seiten 1633-1636, XP055130525, ISSN: 1525-7797, DOI: 10.1021/bm049823x
- EL-MOWAFY A M ET AL.: "Evaluation of renal protective effects of the green-tea (EGCG) and red grape resveratrol: role of oxidative stress and inflammatory cytokines", NATURAL PRODUCT RESEARCH, Bd. 25, Nr. 8, April 2011 (2011-04), Seiten 850-856, XP009179287,

## Beschreibung

Die vorliegende Erfindung betrifft ein Konjugat enthaltend mindestens ein nierenselektives Trägermolekül und mindestens einen Wirkstoff, der eine schützende Wirkung für die Niere vor nephrotoxischen Wirkstoffen aufweist, ein Verfahren zur Herstellung des Konjugats, das Konjugat zur Verwendung zum Schutz der Niere vor nephrotoxischen Wirkstoffen, sowie ein Arzneimittel enthaltend das Konjugat.

Die Niere ist insbesondere für den Transport und die Ausscheidung verschiedener Substanzen und bei der Produktion von Hormonen von Bedeutung. Eine Funktion der Nieren ist die Ausscheidung von Endprodukten des Stoffwechsels, den sogenannten harnpflichtigen Substanzen, und Giftstoffen aus dem Körper durch Bildung des Harns, welcher schließlich über die Harnwege aus dem Körper ausgeschieden wird. Die Niere bilanziert den Wasserhaushalt und dient damit der langfristigen Blutdruckeinstellung. Sie reguliert durch die Kontrolle der Zusammensetzung des Harns den Elektrolythaushalt und den Säure-Basen-Haushalt. Weiterhin ist die Niere ein bedeutendes Organ für den Zwischenstoffwechsel des Körpers (sie betreibt Gluconeogenese). Die Niere produziert Hormone, wie beispielsweise Erythropoetin, für die Blutbildung und ist der Abbauort von Peptidhormonen. Aber auch viele Funktionen der Niere selbst werden durch Hormone gesteuert.

Derzeit leiden ca. 280 Millionen Menschen an chronischen Nierenerkrankungen. Es wurden bereits viele diagnostische und therapeutische Methoden entwickelt. Beispielsweise werden zur Behandlung der Niere bzw. zur Beeinflussung der Nierenfunktion Immunsuppressiva, Zytostatika, Immuntherapeutika, Antiphlogistika, Antibiotika, Virostatika, Antihypertensiva, Urikosurika, oder Diuretika eingesetzt.

Eine Reihe von zugelassenen Wirkstoffen, insbesondere Zytostatika, zeigen als dosislimitierende unerwünschte Nebenwirkung Nephrotoxizität. Beispiele für Substanzen mit nephrotoxischer Wirkung sind Cisplatin, Carboplatin, Gentamicin oder Cyclophosphamid. Beispielsweise schädigt das weitverbreitete Zytostatikum Cisplatin die proximalen Tubuluszellen (PTC) der Nieren, so dass die Dosis bei der Einmalgabe und die Anzahl der Therapiezyklen eingeschränkt sind. Die Schädigung der PTCs wird durch intrazellulären oxidativen Stress hervorgerufen (Matsushima H, et al. 1998, Journal of Laboratory and Clinical Medicine, 131:518-526).
WO 2007/022774 A1 offenbart die Verwendung von bestimmten Peptiden zum Schutz der Nieren vor Cisplatin-induziertem Nierenversagen.

Amifostin ist ein zytoprotektives Arzneimittel, bei dem eine chemo- und radioprotektive Wirkung in einer Vielzahl von Modellorganismen und beim Menschen nachgewiesen wurde.

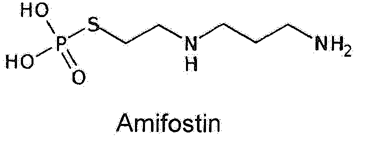

Amifostin selbst ist ein Prodrug, das durch membranständige alkalische Phosphatasen der Endothelzellen in den eigentlichen Wirkstoff 2-((Aminopropyl)amino)ethanthiol gespalten wird.

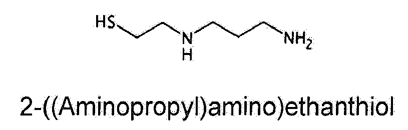

Alkalische Phosphatasen werden in malignem Tumorgewebe wesentlich weniger stark als in gesundem Gewebe exprimiert. Dadurch wird Amifostin hauptsächlich von gesunden Zellen aufgenommen. Diese Selektivität - sie liegt im Bereich von 100:1 - ist notwendig, um die chemo- und radioprotektive Wirkung nicht auch in den Tumorzellen zu entfalten. Die aktive Spezies ist die antioxidative Thiolgruppe von 2-((Aminopropyl)amino)ethanthiol.

Amifostin ist oral nicht verfügbar. Es wird üblicherweise eine halbe Stunde vor einer Radiatio bzw. der Infusion eines Chemotherapeutikums infundiert. Die Dosis liegt dabei im Bereich von 740 bis 900 mg/m² Körperoberfläche. Bei über der Hälfte der Patienten wird dabei eine arterielle Hypotonie als schwerwiegende Nebenwirkung beobachtet.

Es bestand daher der Bedarf die Niere bei einer Behandlung (zum Beispiel einer Radiatio oder einer Infusion eines Chemotherapeutikums) besser und gezielter vor nephrotoxischen Wirkstoffen zu schützen, um die Nebenwirkungen der Wirkstoffe zu vermeiden.

Aufgabe der vorliegenden Erfindung war daher die Bereitstellung einer Lösung, die Niere gezielt vor nephrotoxischen Wirkstoffen zu schützen.

Überraschend wurde gefunden, dass sich Konjugate aus nierenselektiven Trägermolekülen und nierenschützenden Wirkstoffen hervorragend zur Lösung dieser Aufgabe eignen.

Gegenstand der vorliegenden Erfindung ist daher ein Konjugat enthaltend mindestens ein nierenselektives Trägermolekül und mindestens einen Wirkstoff, der eine schützende Wirkung für die Niere vor nephrotoxischen Wirkstoffen aufweist, wie in Anspruch 1 definiert.

Unter einem nierenselektiven Trägermolekül wird erfindungsgemäß ein Molekül verstanden, das als Träger (oder Transporter) für einen Wirkstoff dienen kann und einen zielgerichteten Transport in die Niere ermöglicht. Als Trägermolekül kann eine Verbindung verwendet werden, die nach Konjugation mit dem Wirkstoff eine ausreichend hohe Nierenselektivität aufweist.

Im Stand der Technik sind beispielsweise die folgenden Substanzen bekannt, die sich zum Targeting der Niere, d.h. zum zielgesteuerten Transport in die Niere, eignen:
Kleinere körpereigene Proteine, wie Lysozym (14,3 kDa) können das Glomerulum der Nieren passieren und eignen sich als Transporter für die Adressierung der Nieren mit Wirkstoffen (Franssen et al.: J. Med. Chem. 35, 7, 1992, 1246-1259; Zhang et al.: Biomaterials 30, 2009, S. 1372-1381).

Weiterhin gibt es verschiedene Peptide mit ca. 5 bis 20 Aminosäuren, die selektiv von den Nieren aufgenommen werden. Beispielsweise handelt es sich hierbei um APASLYN und HITSLLS (Denby et al.: Molecular Therapy 15, 9, 2007, 1647-1654) oder ANTPCGPYTHDCPVKR (Kumar und Deutscher: The Journal of Nuclear Medicine 49, 5, 2008, 796-803; Geng et al.: Bioconjugate Chemistry 23, 2012, 1200-1210).

Erdindungsgemäß ist das nierenselektive Trägermolekül ein Peptid, welches zu mehr als 50 % (bezogen auf die Anzahl der Aminosäureeinheiten) aus Sequenzabschnitten ausgewählt aus der Gruppe umfassend -(KKEEE)-, -(RREEE)-, -(KKEE)-, -(KKKEEE)- und -(KKKEE)-besteht,
und wobei
- das Peptid zu mindestens 80 % (bezogen auf die Anzahl der Aminosäureeinheiten) aus den Aminosäuren K und E bzw. R und E besteht,
- und das Peptid 3 bis 5 Sequenzabschnitte wie zuvor definiert enthält.

Unter einem Peptid versteht man erfindungsgemäß eine Verbindung, die aus einer Verknüpfung zweier oder mehrerer Aminosäuren über Amidbindungen entstanden ist. Dabei sind die einzelnen Aminosäuren in einer definierten Reihenfolge (Sequenz) zu einer Kette verbunden. Erfindungsgemäß sind Aminosäuren Verbindungen, die mindestens eine Aminogruppe und mindestens eine Carboxylgruppe tragen. Beispiele sind natürliche, proteinogene Aminosäuren oder nicht-proteinogene in Organismen vorkommende oder synthetisch hergestellte Aminosäuren.

Die Aminosäureeinheiten können im Peptid in der D- oder L-Form vorliegen.
Ein Peptid umfasst zum Beispiel 5 bis 100 Aminosäuren. In einer Ausführungsform weist ein Peptid eine Kettenlänge von 5 bis 40 Aminosäureeinheiten auf, oder eine Kettenlänge von 10 bis 30 Aminosäureeinheiten.

Das Peptid besteht erfindungsgemäß zu mehr als 50% (bezogen auf die Anzahl der Aminosäureeinheiten) aus Sequenzabschnitten wie zuvor definiert.
Bevorzugt besteht es zu mehr als 70 % aus Sequenzabschnitten wie zuvor definiert, besonders bevorzugt zu mehr als 90%.

Bei einer Aminosäure mit saurer Seitengruppe (A) kann es sich beispielsweise um Asparaginsäure, Glutaminsäure, Argininosuccinat und/oder Cysteinsäure handeln. Bevorzugt handelt es sich um Aminosäuren mit Carboxyl-Funktion, das heißt um Glutaminsäure und/oder Asparaginsäure, besonders bevorzugt um Glutaminsäure.

Bei einer Aminosäure mit basischer Seitengruppe (B) kann es sich beispielsweise um Lysin, Arginin, Histidin und/ oder Ornithin handeln. Bevorzugt handelt es sich um Lysin.

Bei einer beliebigen Aminosäure (C) kann es sich beispielsweise handeln um Alanin, Arginin, Asparagin, , Cystein, Glutamin, Glycin, Histidin, Isoleucin, Leucin, Lysin, Methionin, Phenylalanin, Prolin, Serin, Threonin, Tryptophan, Tyrosin, Valin und/oder Citrullin.
Bevorzugt handelt es sich um proteinogene Aminosäuren, die in natürlicher Weise verknüpft sind. Dies gewährleistet den Abbau des Peptids in den proximalen Tubuluszellen der Nieren zu toxikologisch völlig unbedenklichen Metaboliten.

Es werden jeweils die Einbuchstaben-Codes der Aminosäuren benutzt: E (Glutaminsäure), D (Asparaginsäure), C (Cystein), K (Lysin), R (Arginin), H (Histidin).

Erfindungsgemäß steht die Sequenz für eine Sequenz ausgewählt aus der Gruppe umfassend -(KKEEE)-, -(RREEE)-, -(KKEE)-, -(KKKEEE) und - (KKKEE)-.
Besonders bevorzugt steht die Sequenz für die Sequenz -(KKEEE)-:

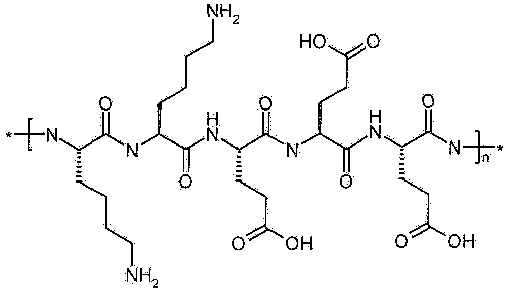

Erfindungsgemäß besteht das Peptid zu mindestens 50 % (bezogen auf die Anzahl der Aminosäureeinheiten) aus den Aminosäuren K und E bzw. R und E. Bevorzugt besteht das Peptid zu mindestens 70 % (bezogen auf die Anzahl der Aminosäureeinheiten) aus den Aminosäuren K und E bzw. R und E, besonders bevorzugt zu mindestens 80 %.

In einer möglichen Ausführungsform enthält das Peptid mehrere direkt aufeinanderfolgende Sequenzabschnitte. Bevorzugt enthält das Peptid 3 bis 5 aufeinanderfolgende Sequenzabschnitte.

Beispielsweise kann das Peptid aus 3 bis 5 aufeinanderfolgenden Sequenzabschnitten sowie einer oder mehreren weiteren Aminosäuren am C- und/ oder N-Terminus bestehen. Dies ist in Formel (2) veranschaulicht:

Xₚ(Sequenzabschnitt)ₓY_{q} (2)

worin der Sequenzabschnitt wie zuvor definiert ist,
x für 3, 4, oder 5 steht,
X und Y unabhängig voneinander für eine beliebige Aminosäure, bevorzugt für A, stehen und
p und q unabhängig voneinander für eine ganze Zahl zwischen 0 und 3 stehen, bevorzugt für 0 oder 1.

Beispiele für mögliche Peptide im erfindungsgemäßen Konjugat sind Peptide ausgewählt aus der Gruppe umfassend (RREEE)₃R, (KKEE)₅K, (KKKEE)₃K, (KKKEEE)₃K und (KKEEE)₃K.

Ein alternatives nierenselektives Trägermolekül ist das ε-Polylysin-Konjugat, wie es in WO 2011/009539 A1 beschrieben ist. Dieses Trägermolekül ermöglicht ebenfalls eine hochselektive Anreicherung in der Niere. Die Verknüpfung der Lysin-Einheiten im Polymer erfolgt über ihre ε-Aminogruppen.

Unter einem Wirkstoff, der eine schützende Wirkung für die Niere vor nephrotoxischen Wirkstoffen aufweist, versteht man erfindungsgemäß einen Wirkstoff, der die Schädigung der proximalen Tubuluszellen reduziert. Beispielsweise handelt es sich hierbei um einen Wirkstoff ausgewählt aus Antioxidantien, Apoptoseinhibitoren, Wirkstoffen mit Einfluss auf den Zellzyklus, Wirkstoffen die die Reparaturmechanismen der Zellen aktivieren, und Kombinationen davon.

Prinzipiell lässt sich die Schädigung der proximalen Tubuluszellen mit Hilfe von diesen Wirkstoffen auf mehreren Ebenen reduzieren:
In der ersten Stufe kann durch die Blockade der Transportmechanismen der proximalen Tubuluszellen die Aufnahme zytotoxischer Verbindungen in das Innere der Zellen verhindert werden. Die Blockade kann durch spezielle Inhibitoren oder aber auch durch ausreichende Mengen des Transportmoleküls selbst erfolgen, das die Rezeptoren der proximalen Tubuluszellen vorrübergehend blockiert. Eine ähnliche Wirkung haben Substanzen, die die Stoffwechselaktivität der proximalen Tubuluszellen reduzieren, beziehungsweise diese Zellen in der G₀-Phase des Zellzyklus verharren lassen.
In der zweiten Stufe können in die Zelle transportierte oder diffundierte nephrotoxische Verbindungen durch "Antidots", die über den Wirkstofftransporter vor der Gabe des nephrotoxischen Wirkstoffes, in das Zellinnere geschleust wurden, unschädlich gemacht werden.
Ziel der dritten Stufe ist es, die Apoptose geschädigter proximaler Tubuluszellen zu unterdrücken. Zytotoxische Substanzen, wie beispielsweise Cisplatin, bewirken im Zellkern Schäden an der DNA. Übersteigt die Schadenshöhe eine bestimmte Schwelle, so wird der programmierte Zelltod, die Apoptose, in der Zelle ausgelöst. Bei Tumorzellen ist dieser Vorgang erwünscht, bei den proximalen Tumorzellen der Nieren jedoch fatal. Es sind einige Substanzen (Apoptoseinhibitoren) bekannt, die in der Lage sind den programmierten Zelltod zu verhindern, beziehungsweise die Schwelle für die Einleitung der Apoptose in der Zelle zu erhöhen.
In der vierten Stufe können Substanzen in die Zellen eingeschleust werden, die die natürlichen Reparaturmechanismen der Zellen aktivieren und so die DNA-Schäden beheben.

### Blockade der Transportmechanismen (Stufe 1):

An die nierenselektiven Trägermoleküle können Wirkstoffe konjugiert werden, die einen Einfluss auf die Transportmechanismen der proximalen Tubuluszellen haben. Durch gezielt gewählte Wirkstoffmoleküle können zelluläre Transporter sowohl auf der apikalen Seite der proximalen Tubuluszellen, das ist die Seite, die im Lumen liegt und mit dem Primärharn in Kontakt ist, als auch auf der basolateralen Seite, das ist die Seite die zu den Blutgefäßen hin ausgerichtet ist, blockiert werden. Transporter der basolateralen Seite der proximalen Tubuluszellen sind für die proximaltubuläre Sekretion von körpereigen Substanzen und Fremdstoffen, wie beispielsweise Arzneimitteln, von großer Bedeutung. Anionische Substanzen werden über den organischen Anionentransporter 1 (OAT1) von den proximalen Tubuluszellen aufgenommen. Kationische Substanzen dagegen über den organischen Kationentransporter 2 (OCT2). Beide Transporter können durch bestimmte Wirkstoffe inhibiert werden. Ein Beispiel für einen Inhibitor von OAT1 ist der Arzneistoff Probenecid (Kurtz A, et al. 2009, Physiologie, ISBN 3-131-51496-5, S. 365). Probenecid kann an ein erfindungsgemäßes Peptid konjugiert werden und nach der glomerulären Filtration über die apikale Seite der proximalen Tubuluszellen aufgenommen werden. Durch einen labilen Linker, beispielsweise eine Estergruppe, mit der die Carboxy-Gruppe von Probenecid mit dem Trägermolekül konjugiert ist, kann der chemisch unveränderte Wirkstoff im Endosom der proximalen Tubuluszellen durch Esterasen freigesetzt werden. Der freie Wirkstoff kann durch Diffusion oder Transporter zur basolateralen Seite gelangen und dort den organischen Anionentransporter 1 blockieren.

Ein Beispiel für einen Inhibitor des organischen Kationentransporters 2 ist der Arzneistoff Tacrin (Sung JH, et al. 2005, Drug Metab Dispos, 33(3):440-448 PMID 15547049). Auch Tacrin lässt sich, beispielsweise als Schiffsche Base oder amidisch, an ein erfindungsgemäßes Peptid konjugieren. Auf dem im Beispiel des konjugierten Probenecids beschriebenen Weg lässt sich so der organische Kationentransporter 2 blockieren. Beispielsweise wird das nierentoxische Zytostatikum Cisplatin von den proximalen Tubuluszellen über OCT2 an der basolateralen Seite aufgenommen und angereichert. In den proximalen Tubuluszellen entfaltet dann das Cisplatin seine nierenschädigende Wirkung (Freissmuth M, et al. 2012, Pharmakologie & Toxikologie, ISBN 3-642-12353-8, S. 735).

### Antioxidantien (Stufe 2):

An die nierenselektiven Trägermoleküle können eine Reihe von antioxidativ wirkenden Substanzen konjugiert werden. Als Wirkstoffklassen kommen unter anderem Polyphenole (Resveratrol, Kaffeesäure, Luteolin, Quercetin, Rutin, Cyanidin, Xanthohumol,...), Liponsäure, Ascorbinsäure, Nicotinsäure, Amifostin, Alliin, Thiole (z.B. 2-Mercaptoethansulfonat-Natrium (Mesna)), Tocopherole, Carotinoide und/ oder Butylhydroxytoluol (BHT), oder Kombinationen hiervon, in Frage.

Wird als nierenselektives Trägermolekül ein ε-Polylysin-Konjugat verwendet, wie es in WO 2011/009539 A1 offenbart ist, kann der molekulare Aufbau des Oligomers derart variiert werden, dass Bausteine der Aminosäure Cystein in das Oligomer eingebaut werden. Cystein hat eine freie Thiolgruppe mit antioxidativer Wirkung. Mit diesem Aufbau kann der Carrier zu einem aktiven, antioxidativ wirkenden Arzneimittel modifiziert werden. Unabhängig davon können an den peptidischen Carrier stattdessen oder zusätzlich eine Reihe von protektiven Wirkstoffen konjugiert werden, wie zuvor beschrieben.

### Apoptoseinhibitoren (Stufe 3):

An den renalen Wirkstofftransporter sind anti-apoptotische Substanzen konjugierbar. Beispiele für diese Wirkstoffgruppe sind Pifithrin-µ (Nijboer et al. 2011, Ann Neurol.:doi: 10.1002/ana.22413) und Pifithrin-α (Komarova et al. 2000, Biochemistry (Mosc) 65(1):41-48) sowie MDL 28170 (Kawamura et al. 2005, Brain Res. 1037(1-2):59-69) und NS3694 (Zhao et al. 2010, Age (Dordr). 32(2):161-177). Pifithrin-α, ein p53-Inhibitor, kann die Schwelle für das Auslösen der Apoptose in behandelten Zellen und Modellorganismen erheblich anheben.

Ein Vorteil der Apoptoseinhibitoren ist, dass sie auch noch nach der Schädigung der Zellen verabreicht werden können. Der Nachteil der systemischen Gabe von Apoptoseinhibitoren, die ggf. ein erhöhtes Krebsrisiko zur Folge haben, kann durch die organspezifische Verabreichung - mit dem erfindungsgemäßen Carrier-Molekül - vorteilhaft verhindert werden.

### Wirkstoffe mit Einfluss auf den Zellzyklus bzw. -metabolismus:

Neben den Antioxidantien und den Apoptoseinhibitoren sind Verbindungen, die einen (vorübergehenden) Stillstand des Zellzyklus der proximalen

Tubuluszellen bewirken, ebenfalls potenzielle Wirkstoffe, mit denen die Schädigung der Nieren durch nephrotoxische Arzneimittel herabgesetzt werden kann. Ein Beispiel hierfür ist die Verbindung Apigenin (Ruela-de-Sousa et al. 2010, Cell death & disease. 1, e19).

### Wirkstoffe, die die Reparaturmechanismen der Zellen aktivieren (Stufe 4):

Durch Aktivierung bestimmter Transkriptionsfaktoren, wie beispielsweise Sp1, oder MDC1 (Luo et al. 2012, The EMBO journal, 31(13):3008-3019) kann eine Zelle zu einer verstärkten Reparatur von (Doppel)-Strangbrüchen angeregt werden (Beishline et al. 2012, Molecular and cellular biology, DOI: 10.1128/MCB.00049-12. PMID 22826432). Das Flavonoid Baicalein (5,6,7-Trihydroxyflavon) ist ein Beispiel für eine Verbindung, die die DNA-Reparatur in Zellen aktivieren kann (Kim et al. 2012, Cell Biol Toxicol, DOI: 10.1007/s10565-012-9233-y. PMID 23011636).
Darüberhinaus kann durch einen Zellzyklusarrest (Stufe 1) die Zelle in einen Zustand versetzt werden, in dem im Wesentlichen "Reparaturarbeiten" an der beschädigten DNA durchgeführt werden (Saleh et al. 2012, Cancer biology & therapy 11, PMID 22895066).

Besonders vorteilhaft ist die (gleichzeitige) Verabreichung mehrerer Wirkstoffklassen (Stufen 1 bis 4 der vorangegangen Beispiele), um die Schädigung der proximalen Tubuluszellen möglichst gering zu halten. Die einzelnen Wirkstoffe können dabei entweder an gesonderte Transportermoleküle oder mehrere unterschiedliche Wirkstoffe an ein Transportermolekül konjugiert werden.

Mögliche Wirkstoffe im Konjugat können daher ausgewählt sein aus der Gruppe umfassend Antioxidantien, Apoptoseinhibitoren, Wirkstoffen mit Einfluss auf den Zellzyklus, Wirkstoffen die die Reparaturmechanismen der Zellen aktivieren, Rezeptorinhibitoren und Kombinationen davon.

In einer bevorzugten Ausführungsform handelt es sich bei Wirkstoff, der eine schützende Wirkung für die Niere vor nephrotoxischen Wirkstoffen aufweist, um ein Antioxidans und/ oder einen Apoptoseinhibitor.

Bevorzugte Antioxidantien sind Liponsäure, Resveratrol, Kaffeesäure, Luteolin, Quercetin, Rutin, Cyanidin, Xanthohumol, Ascorbinsäure, Nicotinsäure, Amifostin, Alliin, Thiole, Mesna, Tocopherole, Carotinoide und Butylhydroxytoluol (BHT).

Bevorzugte Apoptoseinhibitoren sind Pifithrin-µ (Nijboer et al. 2011, Ann Neurol.:doi: 10.1002/ana.22413), Pifithrin-α (Komarova et al. 2000, Biochemistry (Mosc) 65(1):41-48), MDL 28170 (Kawamura et al. 2005, Brain Res. 1037(1-2):59-69) und NS3694 (Zhao et al. 2010, Age (Dordr). 32(2):161-177).

Daher handelt es sich in einer besonders bevorzugten Ausführungsform der vorliegenden Erfindung bei dem Wirkstoff, der eine schützende Wirkung für die Niere vor nephrotoxischen Wirkstoffen aufweist, um Resveratrol, Kaffeesäure, Luteolin, Quercetin, Rutin, Cyanidin, Xanthohumol, Ascorbinsäure, Nicotinsäure, Amifostin, Alliin, Thiole, Tocopherole, Carotinoide, Butylhydroxytoluol (BHT), Pifithrin-µ, Pifithrin-α, MDL 28170 und/oder NS3694, bzw. Mischungen hiervon.

Erfindungsgemäß enthält das Konjugat mindestens ein nierenselektives Trägermolekül, wie zuvor definiert, und mindestens einen Wirkstoff, der eine schützende Wirkung für die Niere vor nephrotoxischen Wirkstoffen, wie zuvor definiert, aufweist.
Die Bindung des Wirkstoffs an das Trägermolekül ist bevorzugt kovalent und kann optional über einen Spacer erfolgen.

Erfindungsgemäß können pro erfindungsgemäßem Konjugat ein oder mehrere gleiche oder unterschiedliche Wirkstoffmoleküle gebunden sein.

Genauso kann das erfindungsgemäße Konjugat, insbesondere bei Makromolekülen wie größeren Wirkstoffmolekülen, beispielweise Proteinen, auch zwei oder mehrere Trägermoleküle enthalten, die an ein Wirkstoffmolekül gebunden sind, um eine nierenspezifische Anreicherung des Wirkstoffs zu ermöglichen. Typischerweise erfolgt auch hier eine kovalente Anbindung der Trägermoleküle an das Makromolekül. Als Makromoleküle gelten erfindungsgemäß nicht nur große Moleküle wie Proteine sondern auch jede Form von Partikeln (z.B. Nanopartikel), Liposomen oder andere Systeme, mit denen Wirkstoffe transportiert oder an die Wirkstoffe gebunden sein können.

Weiterhin können Funktionalitäten für zellspezifisches Targeting wie z. B. Antikörper, Antikörperfragmente oder Aptamere an das erfindungsgemäße Konjugat gebunden sein. Auch Fluoreszenzfarbstofffe oder Interleukine wie IL-2 können gebunden sein.

Die Wirkstoffe oder andere Funktionalitäten können direkt oder mittels eines Spacers kovalent an das Peptid gebunden sein.

Ein Spacer, oft auch als Linker bezeichnet, bewirkt eine kovalente Bindung zwischen zwei Teilen eines Moleküls, im vorliegenden Fall beispielsweise zwischen dem Peptid und einem Wirkstoff. Ein Spacer wird beispielsweise dann eingeführt, wenn die Verbindung zwischen zwei Molekülteilen nicht nur über eine direkte chemische Bindung erfolgen soll, sondern ein gewisser Abstand zwischen zwei Molekülteilen erzeugt werden soll. Genauso kann ein Spacer die chemischen Funktionalitäten zur Verfügung stellen, die notwendig sind, um zwei Teile eines Moleküls, die ansonsten nicht miteinander reagieren würden, zu verbinden. Bevorzugt erfolgt die Konjugation eines Spacers an das Trägermolekül oder einen Wirkstoff über eine Amid- oder eine Esterbindung. Spacer können beispielsweise aliphatische Kohlenwasserstoffe, Polyether (wie Polyethylenglycole), Peptide oder ähnliche Elemente mit Ketten-Struktur sein. Der Spacer kann stabil sein, d.h. er ist unter physiologischen Bedingungen nicht oder nur geringfügig spaltbar, oder er kann instabil sein, d.h. er ist zumindest unter bestimmten physiologischen Bedingungen spaltbar.

Beispiele für funktionelle Gruppen über die eine direkte Anbindung erfolgen kann sind -NH₂, -SH, -OH, -Hal (z.B. -Cl, -Br, -I), -Alkin, -NCS, -NCO, SO₂Cl, -Azid, -Carbonat, -Aldehyd, -Epoxid, -COOH, -COOR, wobei R in diesem Fall bevorzugt ein Halogen oder bevorzugt ein Aktivator, d.h. eine gute Abgangsgruppe ist, z.B. *N*-Hydroxysuccinimid, Pentafluorphenyl oder para-Nitrophenyl. Einen Überblick über mögliche kovalente Kopplungsarten findet man z.B. in "Bioconjugate Techniques", Greg T. Hermanson, Academic Press, 1996 auf den Seiten 137 bis 165.

Beispielsweise können beim erfindungsgemäßen Konjugat Wirkstoffe über einen spaltbaren Linker gebunden sein. Dieser Linker wird dann unter bestimmten Bedingungen, z.B. enzymatisch oder chemisch, *in vivo* gespalten und setzt den Wirkstoff frei. Für diesen Zweck sind Linker, die Carbonsäureester- und Disulfidbindungen enthalten, geeignet, worin die ersteren Gruppen enzymatisch oder chemisch hydrolysiert werden und die letzteren durch Disulfidaustausch, z. B. in Gegenwart von Glutathion, abgetrennt werden.

Ein Beispiel für einen spaltbaren Spacer ist auch ein Peptid, welches gezielt mit Hilfe von speziellen, körpereigenen oder aber auch dem Körper zugesetzten Enzymen gespalten werden kann. So wird z.B. die Peptidsequenz DEVD (Asp-Glu-Val-Asp) nach Apoptoseinduktion durch Caspase-3 gespalten. Damit kann beispielsweise ein Wirkstoff der über einen derartigen Spacer gebunden ist, nach einer bestimmten Verweilzeit in der Niere aus selbiger entfernt werden, oder aber auch eine entsprechende Funktionalität (Vorhandensein oder Abwesenheit eines bestimmten Enzyms) der Niere überprüft werden. Weitere Beispiele sind die Peptidsequenzen CPEN↓FFWGGGG (Salinas et al. 2008, Biomaterials 29, 2370-2377) oder PENFF, die durch die Matrix-Metalloprotease-13 gespalten werden kann.

Eine einfache Ausführungsform eines spaltbaren Spacers ist die Bildung eines Carbonsäureesters, der durch Esterasen leicht gespalten werden kann.
Daher ist in einer bevorzugten Ausführungsform der vorliegenden Erfindung der Wirkstoff über eine Esterverknüpfung gebunden. Dies ermöglicht eine exakte Abspaltung des Wirkstoffmoleküls in der Niere. Gleichzeitig ist die Verknüpfung jedoch zuvor für den Transport in die Niere ausreichend stabil, um eine vorzeitige Abspaltung zu vermeiden.
Weiterhin ermöglicht eine leicht spaltbare Esterverknüpfung des Wirkstoffes mit dem Wirkstofftransporter, eine relativ schnelle Freisetzung des Wirkstoffs am Zielort. Die Spaltung der Esterverknüpfung erfolgt zeitlich schneller als der Abbau des Wirkstofftransporters durch Proteasen.

Alternativ kann der Spacer eine säurelabile Struktur, z.B. ein Hydrazon, ein Imin, ein Carboxylhydrazon, ein Acetal oder Ketal enthalten (siehe beispielsweise Haag-R, Kratz-F, Angewandte Chemie Seite 1218 (2006)).

Erfindungsgemäß kann der mindestens eine Wirkstoff an den N- und/oder C-Terminus des Trägermoleküls gebunden sein.
In einer alternativen Ausführungsform kann der Wirkstoff an eine Aminosäure in der Kette gebunden sein.
In einer weiteren alternativen Ausführungsform kann der Wirkstoff in der Kette zwischen den Aminosäuren gebunden sein.

Das erfindungsgemäße Konjugat wird hochselektiv von den Nieren aufgenommen und relativ rasch abgebaut.
Durch die geeignete Auswahl des Verknüpfungsortes des Wirkstoffs am Trägermolekül, sowie durch die geeignete Auswahl der Kettenlänge und des molekularen Aufbaus des Trägermoleküls, lässt sich dabei die gewünschte Pharmakokinetik, d.h. die gewünschte Wirkstofffreisetzung am Zielort, d.h. in der Niere, einstellen.

Typischerweise führen längere Trägermoleküle zu einer verzögerten Freisetzung verglichen mit kürzeren Trägermolekülen. Längere Trägermoleküle haben beispielsweise Kettenlängen von 20 bis 40 Aminosäuren, bevorzugt 30 Aminosäuren, während unter kürzeren Trägermolekülen typischerweise Kettenlängen von 3 bis 10 Aminosäuren, bevorzugt 5 Aminosäuren, verstanden werden.

Die Freisetzung C-terminal-verknüpfter Wirkstoffe erfolgt wesentlich schneller als die von N-terminal-verknüpften Wirkstoffen. Ohne an diese Theorie gebunden zu sein, wird vermutet, dass der geschwindigkeitsbestimmende Schritt beim Peptidabbau vor allem durch Carboxypeptidasen beeinflusst wird, die vom C-Terminus aus beginnend das Peptid abbauen.

Erfindungsgemäß werden auch verzweigt in der Kette eingebaute Wirkstoffe deutlich langsamer freigesetzt, als linear verknüpfte. Der enzymatische Abbau verzweigter Peptidstrukturen ist grundsätzlich deutlich schwieriger als der Abbau linearer Peptide.

Weiterhin lässt sich erfindungsgemäß die Freisetzungsgeschwindigkeit des Wirkstoffs auch durch die Art dessen Verknüpfung an das Oligomer steuern. Eine leicht spaltbare Esterverknüpfung ermöglicht eine relativ schnelle Freisetzung des Wirkstoffs am Zielort (siehe oben).

Gegenstand der vorliegenden Erfindung ist auch ein Verfahren zur Herstellung eines Konjugats, wie zuvor beschrieben, dadurch gekennzeichnet, dass ein optional aktivierter Wirkstoff, der eine schützende Wirkung für die Niere vor nephrotoxischen Substanzen aufweist, an das Trägermolekül konjugiert wird.

Die Herstellung der erfindungsgemäßen Konjugate weist typischerweise zumindest die folgenden Verfahrensschritte auf:
a) Bereitstellen eines Trägermoleküls, wie zuvor definiert, das zumindest eine reaktive Gruppe aufweist,
b) Konjugation mindestens eines optional aktivierten Wirkstoffs, wie zuvor definiert, an das Trägermolekül aus Schritt a).

Die Trägermoleküle der erfindungsgemäßen Konjugate können insbesondere auf verschiedene, dem Fachmann im Bereich der Peptidsynthese bekannte, Verfahren hergestellt werden.

Typischerweise erfolgt die Herstellung über eine Festphasensynthese. Eine Festphase ist erfindungsgemäß ein organisches, anorganisches oder organisch/anorganisches Verbundmaterial, das als Harz oder Träger in der Festphasensynthese eingesetzt werden kann. Des Weiteren gelten erfindungsgemäß als Festphase auch Oberflächen von Formkörpern wie z.B. Mikrotiterplatten oder partikuläre Materialien, wie z.B. organische oder anorganische Nanopartikel, Metallpartikel oder ähnliches.

Die Festphasensynthese wird entsprechend einer herkömmlichen Peptid-Synthese durchgeführt (z.B. Fmoc/tBu-Peptid-Synthese oder Boc/Benzyl-Peptid-Synthese). Dem Fachmann sind derartige Festphasensynthesen bekannt. Geeignete Lehrbücher für die Peptidsynthese sind "Solid-Phase Peptide Synthesis": 289 (Methods in Enzymology) von Sidney P. Colowick (Autor), Gregg B. Fields (Herausgeber), Melvin I. Simon (Herausgeber) Academic Press Inc (November 1997) oder "Fmoc Solid Phase Peptide Synthesis: A Practical Approach" von W. Chan (Autor), W. C. Chan (Herausgeber), Peter D. White (Herausgeber) Oxford Univ Pr (2. März 2000). Die jeweils eingesetzten Monomere werden dabei so gewählt, dass ein Peptid entsprechend der vorliegenden Erfindung entsteht. Je nach Art der Aminosäureeinheit kann zur Synthese direkt eine derivatisierte Aminosäureeinheit verwendet werden oder eine zunächst an der für die Derivatisierung vorgesehenen Stelle geschützte Aminosäureeinheit. Nach erfolgter Synthese des Peptids kann dann entweder noch an der Festphase oder nach Abspaltung von der Festphase in Lösung die abschließende Derivatisierung mit dem Wirkstoff vorgenommen werden.

Die Anbindung des Wirkstoffs erfolgt in diesem Fall bevorzugt an das fertige Peptid, d.h. entweder nach erfolgter Festphasensynthese des Peptids noch an der Festphase oder nach dessen Abspaltung in Lösung.

Soll der Wirkstoff beispielsweise an das N-terminale Ende des Peptids gebunden werden, werden die Peptide typischerweise mit einer Aminoterminalen Schutzgruppe erzeugt, wie etwa Fmoc. Sofern der Wirkstoff die Bedingungen überstehen kann, die verwendet werden, um einerseits das Peptid vom Syntheseharz abzuspalten und andererseits die Seitenketten zu entschützen, kann die Fmoc-Gruppe vom N-Terminus des vollständigen Harz-gebundenen Peptids abgespalten werden, so dass der Wirkstoff an das freie N-terminale Amin gebunden werden kann. In solchen Fällen wird der Wirkstoff typischerweise durch Verfahren aktiviert, die in der Technik allgemein dafür bekannt sind, dass sie eine aktive Ester- oder aktive Carbonatgruppe erzeugen, die wirkungsvoll ist, um eine Amid- bzw. Carbamatbindung mit der Oligomer-Aminogruppe zu bilden. Natürlich kann auch eine andere Verknüpfungschemie verwendet werden.

Um Nebenreaktionen zu minimieren, können Guanidino- und Amidinogruppen unter Verwendung herkömmlicher Schutzgruppen, wie etwa Carbobenzyloxygruppen (CBZ), di-*t*-BOC, PMC, Pbf, N-NO₂ und ähnlichen blockiert werden.

Kopplungsreaktionen werden durchgeführt durch bekannte Kopplungsverfahren in Lösungsmitteln, wie etwa N,N-Dimethylformamid (DMF), *N*-Methylpyrrolidon (NMP), Dichlormethan (DCM) und/oder Wasser. Beispielhafte Kopplungsreagenzien umfassen O-Benzotriazolyloxytetramethyluroniumhexafluorphosphat (HATU), Dicyclohexylcarbodiimid, Brom-tris(pyrrolidino)phosphoniumbromid (PyBroP), usw. Andere Reagenzien können enthalten sein, wie etwa *N,N-*Dimethylaminopyridin (DMAP), 4-Pyrrolidinopyridin, *N*-Hydroxysuccinimid oder N-Hydroxybenzotriazol (HOBt).

Ein Trägermolekül basierend auf einem ε-Polylysin-Konjugat, wie in WO 2011/009539 A1 beschrieben, kann auch ausgehend von ε-Polylysin hergestellt werden. Dabei wird typischerweise synthetisches oder natürliches ε-Polylysin einheitlicher oder unterschiedlicher Kettenlänge in Lösung mit den entsprechenden Carboxylgruppen tragenden Verbindungen zur Reaktion gebracht. Dazu können beispielsweise zunächst die Carboxylgruppen tragende Verbindungen aktiviert werden. Dies kann z.B. durch Aktivierung einer oder mehrerer ihrer Carboxylgruppen erfolgen, indem sie zum Aktivester oder Säurechlorid umgesetzt werden. Anschließend erfolgt die Umsetzung mit ε-Polylysin, wobei die Konjugation bevorzugt an die freien Aminogruppen erfolgt. Alternativ können beispielsweise ein oder mehrere Carboxylgruppen der Carboxylgruppen tragenden Verbindung mit einem Kopplungsreagenz wie Dicyclohexylcarbodiimid (DCC) oder HATU aktiviert und mit dem ε-Polylysin umgesetzt werden, wobei die Konjugation bevorzugt an die freien Aminogruppen erfolgt. Reaktionsbedingungen für derartige Umsetzungen sind dem Fachmann bekannt. Geeignete Lösungsmittel sind beispielsweise Wasser, Acetonitril, DMSO, DMF, Dioxan, THF, Methanol oder Mischungen zweier oder mehrerer der genannten Lösungsmittel.

Die erfindungsgemäßen Konjugate haben den Vorteil, dass systemische Nebenwirkungen von Wirkstoffen zur Behandlung oder Bildgebung der Niere weitgehend unterdrückt werden können, da die Konjugate einen gezielten Transport von nierenschützende Substanzen in die Niere ermöglichen. Zu diesen nierenschädigenden Wirkstoffen gehören beispielsweise Cisplatin, Carboplatin, Gentamicin und Cyclophosphamid. Bei diesen Substanzen ist die Nephrotoxizität dosislimitierend bzw. limitiert sie die Anzahl der Therapiezyklen.
In Zusammenhang mit der Behandlung der Niere mit nierenschädigen Substanzen ermöglicht die Verabreichung der erfindungsgemäßen Konjugate (z.B. durch Injektion in die Blutbahn oder nach subkutaner Injektion) vor, während oder nach der Therapie eine gezielte Anreicherung der schützenden Wirkstoffe in der Niere.
Der mit einer nierenschädigenden Substanz, beispielsweise Cisplatin, behandelte Patient erhält etwa 30 Minuten vor der dem Beginn der Therapie eine Injektion des nierenschützenden Konjugates. Das nierenschützende Konjugat kann dabei per Einmalinjektion oder durch kontinuierliches Infundieren über den gesamten Zeitraum der Cisplatin-Gabe, die sich üblicherweise über einen Zeitraum von ein bis zwei Stunden erstreckt, erfolgen.

Gegenstand der vorliegenden Erfindung ist daher auch ein erfindungsgemäßes Konjugat, wie zuvor beschrieben, als Arzneimittel, wie insbesondere einer therapeutischen Zusammensetzung, insbesondere zum Schutz der Niere vor nephrotoxischen Wirkstoffen.

Gegenstand der vorliegenden Erfindung ist auch ein erfindungsgemäßes Konjugat, wie zuvor beschrieben, zur Verwendung zum Schutz der Niere vor nephrotoxischen Wirkstoffen.

In diesem Zusammenhang ist die Verwendung eines Trägermoleküls, wie zuvor definiert, besonders vorteilhaft, da sich dieses außerordentlich gut zum Targeting der Niere eignet: Im Vergleich zu anderen bekannten niedermolekularen Strukturen zeigt es auch in der Konjugation mit dem Wirkstoff eine sehr gute Nierenanreicherung. Der Vergleich mit in der Literatur beschriebenen Peptiden, die selektiv von den Nieren aufgenommen werden (APASLYN und HITSLLS, Aminosäuren sind im Einbuchstaben-Code angegeben (Denby et al.: Molecular Therapy 15, 9, 2007, 1647-1654)), ergibt, dass zwar die meisten Peptide eine mehr oder minder stark ausgeprägte Nierenselektivität nach der intravenösen Applikation aufweisen, jedoch nicht in der Konjugation mit einem Wirkstoff. Der pharmakologische Nutzen der Peptidstrukturen als Transportsystem für den zielgerichteten Schutz der Niere vor nephrotoxischen Substanzen ist jedoch nur dann gegeben, wenn diese Peptide zusammen mit konjugierten Wirkstoffen nahezu ausschließlich von den Nieren, namentlich den proximalen Tubuluszellen, aufgenommen werden. Nur so ergibt sich ein signifikanter Vorteil gegenüber der systemischen Gabe des Wirkstoffes.

Weiterhin ermöglichen die erfindungsgemäßen Konjugate, dass neben der in der Literatur beschriebenen intravenösen Gabe von Peptiden/Proteinen zum Wirkstofftransport in die Nieren auch die subkutane und intraperitoneale Gabe der erfindungsgemäßen Peptid-Wirkstoff-Konjugate erfolgreich die Nieren adressieren kann.
Die intraperitonielle, und speziell die subkutane Administrationsroute, ist für die Applizierung eines potentiellen Wirkstoffes, gegenüber der intravenösen Route, für Arzt und Patient vorteilhaft.

Gegenstand der vorliegenden Erfindung ist auch ein Arzneimittel bzw. eine pharmazeutische Zusammensetzung, insbesondere eine therapeutische oder eine bildverstärkende Zusammensetzung, enthaltend mindestens ein erfindungsgemäßes Konjugat, wie zuvor beschrieben.

Erfindungsgemäß kann das Konjugat auch in Form seiner pharmazeutisch verwendbaren Salze und Stereoisomere, einschließlich deren Mischungen in allen Verhältnissen, vorliegen.

Erfindungsgemäß ist auch die Verwendung der erfindungsgemäßen Konjugate zur Herstellung einer pharmazeutischen Zusammensetzung bzw. eines Arzneimittels, insbesondere einer therapeutischen Zusammensetzung.

Erfindungsgemäß kann es sich bei der vorliegenden Erfindung auch um ein Kit zur Herstellung eines Arzneimittels bzw. einer pharmazeutischen Zusammensetzung handeln, insbesondere einer therapeutischen Zusammensetzung, enthaltend mindestens ein erfindungsgemäßes Konjugat. Dieses Konjugat kann dann je nach Anwendungszweck beispielsweise mit einem geeigneten Wirkstoff umgesetzt werden zur Herstellung einer therapeutischen Zusammensetzung.

Die vorliegende Erfindung betrifft zudem die erfindungsgemäßen Konjugate, und/oder deren pharmazeutisch verwendbaren Salze und Stereoisomere, einschließlich deren Mischungen in allen Verhältnissen, sowie gegebenenfalls Träger- und/oder Hilfsstoffe
- als Arzneimittel
- zur Verwendung als Arzneimittel
- als Wirkstoff oder wirksame Komponente in einem Arzneimittel
- zur Verwendung zum Schutz der Niere vor nephrotoxischen Wirkstoffen
- sowie insbesondere als Arzneimittel zum Schutz der Niere vor nephrotoxischen Wirkstoffen.

Eine therapeutische Zusammensetzung, eine pharmazeutische Zusammensetzung oder ein Arzneimittel besteht in der Regel zumindest aus dem Wirkstoff - in diesem Fall dem erfindungsgemäßen Konjugat mit dem angebundenem Wirkstoff - und einem oder mehreren geeigneten Lösungsmitteln und/oder Trägerstoffen, die die Applikation der therapeutischen Zusammensetzung erlauben.

Pharmazeutische Zusammensetzungen bzw. Arzneimittel oder Medikamente lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste pharmazeutische Formulierungen können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Calciumstearat oder Polyethylenglykol in Festform können dem Pulvergemisch vor dem Füllvorgang zugesetzt werden. Ein Sprengmittel oder Lösungsvermittler, wie z.B. Agar-Agar, Calciumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glucose oder β-Lactose, Süßstoffe aus Mais, natürliche und synthetische Gummi, wie z.B. Akazia, Traganth oder Natriumalginat, Carboxymethylcellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylcellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylcellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dicalciumphosphat, vermischt wird. Das Pulvergemisch lässt sich granulieren, indem es mit einem Bindemittel, wie z.B. Sirup, Stärkepaste, Acadia-Schleim oder Lösungen aus Cellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die erfindungsgemäßen Konjugate lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Konjugate können auch unter Verwendung monoklonaler Antikörper als individuelle Träger, an die die Konjugate gekoppelt werden, zugeführt werden. Die Konjugate können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Poly-s-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste pharmazeutische Verbindungen können als Salben, Cremes, Suspensionen, Lotionen, Pulver, Lösungen, Pasten, Gele, Sprays, Aerosole oder Öle formuliert sein.

An die rektale Verabreichung angepasste pharmazeutische Formulierungen können in Form von Zäpfchen oder Einläufen dargereicht werden.

An die nasale Verabreichung angepasste pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

Zu den an die parenterale Verabreichung angepassten pharmazeutischen Formulierungen gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Bevorzugt werden die erfindungsgemäßen Konjugate parenteral verabreicht.

Es versteht sich, dass die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge des erfindungsgemäßen Konjugates hängt von einer Reihe von Faktoren ab, einschließlich Art des gekoppelten Wirkstoffs, dem Alter und Gewicht des Patienten, dem exakten Krankheitszustand, der der Behandlung bedarf, sowie seines Schweregrads, der Beschaffenheit der Formulierung sowie dem Verabreichungsweg.

Gegenstand der vorliegenden Erfindung ist auch ein Kit zur Herstellung einer pharmazeutischen Zusammensetzung, insbesondere einer bildverstärkenden oder therapeutischen Zusammensetzung, zumindest enthaltend ein erfindungsgemäßes Konjugat. Das erfindungsgemäße Konjugat kann in dem Kit in einem Lösungsmittel (z.B. einem wässrigen Puffer) gelöst vorliegen oder bevorzugt als Lyophilisat.

Es wurde gefunden, dass die erfindungsgemäßen Konjugate bereits kurze Zeit nach der Applikation spezifisch, d.h. ausschließlich bzw. fast ausschließlich in der Niere angereichert werden. Im Falle der bevorzugten intravenösen Gabe der erfindungsgemäßen Konjugate ist schon nach 5 Minuten eine Anreicherung in der Niere zu beobachten. Nach einer Stunde befinden sich mehr als 30%, bevorzugt mehr als 50%, besonders bevorzugt mehr als 70%, ganz besonders bevorzugt mehr als 80% der injizierten Dosis in der Niere (%-Angaben basieren auf Messung der Radioaktivität).

Bei Organverteilungsstudien mit radiomarkierten erfindungsgemäßen Konjugaten (beispielsweise PET-Messungen oder anderer nicht-invasiver Bildgebung) zeigen die erfindungsgemäßen Konjugate eine Stunde nach intravenöser Applikation typischerweise mindestens eine 2-fache, bevorzugt mindestens eine 5-fache, besonders bevorzugt mindestens eine 10-fache Anreicherung in der Niere im Verhältnis zum übrigen Körper (Blut, Herz, Lunge, Milz, Leber, Muskel, Gehirn). Das bedeutet, dass das Signal, das direkt mit der Menge der radiomarkierten Verbindung korreliert, in der Niere mindestens 2 mal stärker ist als die Summe der von Blut, Herz, Lunge, Milz, Leber, Muskel und Gehirn zusammen erhaltenen Signale.

Targeting der Niere bedeutet erfindungsgemäß das Erreichen einer vermehrten Aufnahme des applizierten Stoffes in der Niere im Verhältnis zum übrigen Körper. Beim Targeting der Niere mit dem erfindungsgemäßen Konjugat wird durch Gabe eines erfindungsgemäßen Konjugats bevorzugt mindestens eine 2-fache, bevorzugt mindestens eine 5-fache, besonders bevorzugt mindestens eine 10-fache Anreicherung in der Niere im Verhältnis zum übrigen Körper (Blut, Herz, Lunge, Milz, Leber, Muskel, Gehirn) erreicht. Diese Werte werden mittels Organverteilungsstudien mit radiomarkierten erfindungsgemäßen Konjugaten (beispielsweise PET-Messungen oder anderer nicht-invasiver Bildgebung) ermittelt. Die Anreicherung in der Niere erfolgt je nach Art der Applikation typischerweise nach 30 Minuten bis 8 Stunden.

### Abbildungen:

Abb. 1 zeigt den Einfluss der Kettenlänge auf die Wirkstofffreisetzung für die Strukturen MAG3-KKEEEKKEEEKKEEEK und MAG3-KKEEEKKEEEKKEEEKKEEEKKEEEKKEEE (N-terminale Verknüpfung des Wirkstoffs - Abb. 1, oben) und KKEEEKKEEEKKEEE-y und KKEEEKKEEEKKEEEKKEEEKKEEEKKEEE-y (C-terminale Verknüpfung des Wirkstoffs - Abb. 1, unten).
Abb. 2 zeigt den Einfluss der Kettenlänge auf die Wirkstofffreisetzung für die Struktur y-KKEEEKKEEEKKEEEK (N-terminale Verknüpfung des Wirkstoffs -Abb. 2, unten) und die Strukturen KKEEEKKEEEKKEEE-y und KKEEEKKEEEKKEEEKKEEEKKEEEKKEEE-y (C-terminale Verknüpfung des Wirkstoffs -Abb. 2, oben).
Abb. 3 vergleicht die Organverteilung des 125Iod markierten Konjugates y(KKEEE)₃K in Abhängigkeit von der Administrationsroute.
Abb. 4 zeigt die szintigraphische Verteilung des N-terminal gebundenen Diacetylkaffeesäure- (KKEEE)₃K Wirkstoffkonjugats nach intravenöser Applikation in einer NMRI-Maus.
Abb. 5 zeigt die szintigraphische Verteilung des zweifach konjugierten Moleküls yKKK(DCA)EEEKKEEEKKK(DCA)EEEK (CDA=Diacetylkaffeesäure) nach intravenöser Applikation in einer NMRI-Maus.
Abb. 6 zeigt die szintigraphische Verteilung von ¹²⁵I-y(KKKε(Liponsäure)EEE)₃K nach intravenöser Applikation in einer NMRI-Maus.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### 1. Materialsynthesen

### 1.1. Synthese der Peptide mit sauren und basischen Seitengruppen

### Festphasenpeptidsynthese

Die Peptide werden am vollautomatischen Peptidsynthesizer ABI 433A der Firma Applied Biosystems GmbH (Carlsbad, CA, USA) nach der Fmoc/fBu-Strategie unter Verwendung von Tentagel S RAM Harz (Beladungsgrad: 0.24 mmol/g; Rapp Polymere, Tübingen, Deutschland) als polymerer Träger hergestellt. Fmoc-Aminosäuren (Fmoc-AS-OH; Novabiochem, Merck KGaA, Darmstadt, Deutschland) mit säurelabilen Seitenkettenschutzgruppen (z. B. Arg(Pbf), Asn(Trt), Asp(*t*Bu), Cys(Trt), Gln(Trt), Glu(*t*Bu), His(Trt), Lys(Boc), Ser(*t*Bu), Thr(*t*Bu), Tyr(*t*Bu)) werden als Ausgangsmaterialien verwendet. Der Synthesecyclus besteht aus a) Abspaltung der Fmoc-Schutzgruppe mit 20%igem Piperidin in NMP, b) Waschschritte mit NMP, c) Kupplung: Fmoc-AS-OH/HBTU/DIPEA/Peptidharz 10/10/20/1, 8 Min, d) Waschschritte mit NMP. Die Effektivität der Fmoc-Abspaltungen werden mittels automatisierten Leitfähigkeitsmessungen kontrolliert. Die Peptide werden mit TFA/H₂O/Triisopropylsilan (95 : 2,5 : 2,5) vom Harz abgespalten (2 h bei Raumtemperatur), in kaltem Methyl-tert.Butylether ausgefällt, mittels Zentrifugation (4000 rpm, 5 min) aufgetrennt, im Vakuum getrocknet und aus MeCN/H₂O (1 : 1) lyophilisiert.

### Reinigung und Charakterisierung von Peptiden

Die Reinigung des vom Harz abgespaltenen Peptids mittels semi-präparativer HPLC wird mit einer LaPrep-Anlage (VWR GmbH, Darmstadt, Deutschland) durchgeführt. Als Säule wird eine Waters XBridge BEH130 PREP C18 (5 µm, 19 x 150 mm) Säule verwendet (Flussraten: 9 - 20 ml/min; Lösungsmittel: 0,1 % TFA in Wasser zu 0,1 % TFA in Acetonitril). Zur Trennung wird ein Gradient von Wasser auf Acetonitril verwendet, der den physikochemischen Eigenschaften der entsprechenden Peptide angepasst wird. Das aufgereinigte Peptid wird nach Lyophilisierung erhalten.

Zur Charakterisierung werden die hergestellten Peptide mittels analytischer HPLC (Agilent 1100) und HPLC-MS (Exactive, Thermo Fisher Scientific) analysiert. Die HPLC-Analyse unter Standardbedingungen erfolgt anhand eines linearen Gradienten von 0,1 % TFA in Wasser auf 0,1 % TFA in Acetonitril in 5 min (Bedinungen: ChromolithR Performance RP-18e-Säule, 100 x 3 mm; Flussrate: 2 ml/min, Wellenlänge = 214 nm). Für die Massenspektrometrie dient ein Agilent 1200 als HPLC-System (Bedingungen: Hypersil Gold C18 Säule, 0.21 x 200 mm, Gradient: von 0,05 % TFA in Wasser auf 0,05 % TFA in Acetonitirl in 30 min, Flussrate: 200 µl/min, Säulenofen: 60 °C, Wellenlänge = 214 nm).

### Radioaktive Iodierung von Peptiden

Zur Markierung wird eine 1 mM Stammlösung des zu markierenden Peptids in Wasser (evtl. muss DMSO zur besseren Löslichkeit zugesetzt werden) verwendet. Tyrosin-haltige Peptide werden mit lod-123, Iod-125 oder lod-131 mittels der Chloramin-T-Methode markiert (Perkin-Elmer, Waltham, MA, USA). Dazu werden 10 µl der Stammlösung mit 20 µl Phosphatpuffer (0,25 M, pH 7,4) versetzt und die gewünschte Menge an radioaktivem lod zugegeben. Zur Markierung werden 5 µl Chloramin-T (2 mg/ml H₂O) hinzugefügt. Die Reaktion erfolgt für 30 Sekunden und wird anschließend mit 10 µl einer gesättigten Methionin-Lösung abgebrochen. Um freies lod und Nebenprodukte abzutrennen, wird das Reaktionsgemisch mittels semi-präparativer HPLC (Chromolith RP-18e, 100 x 4.6 mm) gereinigt. Zur Trennung wird ein linearer Gradient von 0,1 % TFA in Wasser auf 0,1 % TFA in Acetonitril in 10 Minuten verwendet (Flussrate: 2 ml/min, UV-Absorption bei 214 nm, γ-Detektion). Anschließend wird das Lösungsmittel am Rotationsverdampfer entfernt und das markierte Peptid im gewünschten Puffer aufgenommen.

### 1.2. Herstellung von Liponsäure-y(KKEEE)₃K

Das Peptid y(KKEEE)₃K wird wie unter 1.1 beschrieben mittels Festphasensynthese der Fmoc/*t*Bu-Strategie unter Verwendung der Aminosäuren Fmoc-Lys(Boc)-OH, Fmoc-Glu(O*t*Bu)-OH und Fmoc-Tyr(*t*Bu)-OH (Novabiochem, Merck KGaA, Darmstadt, Deutschland) am Peptidsynthesizer hergestellt. Das Peptid wird zunächst nicht vom Harz abgespalten, sondern nach der finalen Fmoc-Entschützung in NMP suspendiert (pro 100 mg Peptidharz werden 1 ml NMP verwendet). (RS)-Liponsäure (Merck KGaA, Darmstadt, Deutschland; 4 Äquivalente bezogen auf die Harzbeladung) wird währenddessen in NMP (1 ml pro 100 mg) gelöst, mit HBTU (4 Äq.) versetzt und bei Raumtemperatur für ca. 10 min gerührt. Das Reaktionsgemisch wird zum Peptidharz addiert, mit DIPEA (10 Äq.) versetzt und bei Raumtemperatur für ca. 4 h geschüttelt. Das Harz wird 5 x mit NMP und 5 x mit DCM gewaschen und im Vakuum für ca. 4 h getrocknet. Das Liponsäure-Peptid-Konjugat wird mit TFA/Thioanisol/EDT/Anisol (90/5/3/2) für ca. 1 h bei Raumtemperatur vom Harz abgespalten, in kaltem Methyl-tert.-Butylether ausgefällt, mittels Zentrifugation (4000 rpm, 5 min) aufgetrennt, im Vakuum getrocknet, aus MeCN/H₂O (1 : 1) lyophilisiert und wie unter 1.1 Reinigung und Charakterisierung der Peptide beschrieben aufgereinigt.

Auf analoge Weise lassen sich auch Konjugate mit anderen Wirkstoffen herstellen.

### 1.3 Herstellung von

### yKKK(Diacetylkaffeesäure)(EEEKK)₂K(Diacetylkaffeesäure)EEEK

Für die peptische Konjugation von Diacetylkaffeesäure an eine Lysinseitenkette wird die Aminosäure Fmoc-Lys(Mmt)-OH in die Sequenz des Peptidrückgrats eingebaut. Das wie unter 1.1 hergestellte Peptid-Harz wird dabei vor der Abspaltung 3 min mit Dichlormethan (DCM)/Triisopropylsilan/TFA (94 : 5 : 1) versetzt und 5 x mit DCM gewaschen. Dieser Vorgang wird 3 x wiederholt. Für die Kupplung an die orthogonalentschützte Seitenkette von Lysin werden 4 äq der Diacetylkaffeesäure in NMP gelöst, mit 4 äq 1-Ethyl-3-(3-dimethylaminopropyl)-carbodiimid (EDC), 4 äq Ethylcyano(hydroxyimino)acetate (Oxyma Pure) und 10 äq Diisopropylethylamin (DIPEA) versetzt, bei Raumtemperatur für ca. 10 min gerührt und anschließend zum Peptid-Harz addiert. Das Reaktionsgemisch wird für ca. 1 h bei Raumtemperatur geschüttelt, 5 x mit NMP und 5 x mit DCM gewaschen und im Vakuum getrocknet. Das funktionalisierte Peptid wird wie unter 1.1 beschrieben vom Harz abgespalten und aufgereinigt.

Auf analoge Weise lassen sich auch Konjugate mit anderen Wirkstoffen herstellen.

### 1.4 Herstellung von y(KKKε(Liponsäure)EEE)₃K

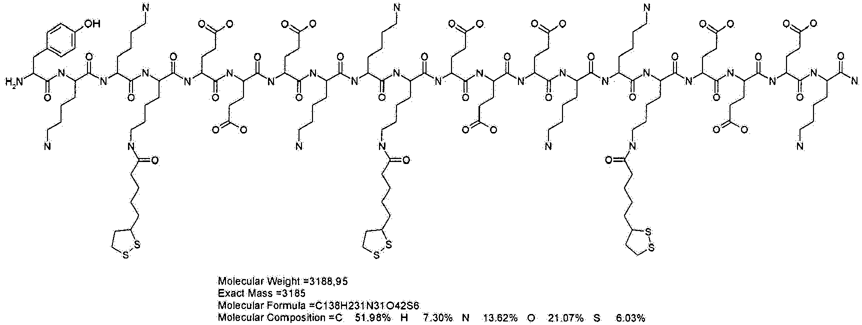

### 1.4.1 Synthese des Fmoc-Lysin(ε-Liponsäure)-OH-Bausteins

N-Hydroxysuccinimid (1,15 g, 10 mmol), α-Liponsäure (2,02 g, 9,8 mmol) und (1,92 g, 10 mmol) 1-Ethyl-3-(3-dimethylaminopropyl)carbodiimid (EDAC) werden in 50 ml DMF gelöst und bei Raumtemperatur für ca. 4 h gerührt. Danach wird der Ansatz mit 60 ml Ethylacetat versetzt. Die organische Phase wird dreimal mit 60 ml destilliertem Wasser, dreimal mit 60 ml gesättigter Natriumhydrogencarbonat-Lösung und einmal mit gesättigter Natriumchlorid-Lösung gewaschen. Die Ethylacetat-Phase wird über Na₂SO₄ getrocknet, filtriert und bis zur Trockene eingeengt.
Ausbeute: 2,23 g (73,5 %)

Fmoc-Lys-OH (2,65 g, 7,2 mmol) wird in 110 ml HEPES-Puffer (pH= 7,4) suspendiert und mit (2,14 g, 7,05 mmol) Liponsäureaktivester (gelöst in 130 ml Acteon) versetzt und bei Raumtemperatur gerührt. Nach ca. 3 h Reaktionszeit wird die Lösung mittels 0,1 N NaOH-Lösung auf pH 7 eingestellt und bei Raumtemperatur für ca. 20 h gerührt. Danach wird der Ansatz mit 0.1 N NaOH auf pH 9 gebracht und zweimal mit ca. 30 ml Ethylacetat gewaschen, anschließend wird mit 1 N HCl auf pH 3 gestellt und dreimal mit ca. 40 ml Ethylacetat extrahiert. Die vereinigten org. Phasen werden mit gesättigter Natriumchlorid-Lösung gewaschen, über Na2SO4 getrocknet, filtriert und bis zur Trockene eingeengt.
Auswaage Rohprodukt: 4,14 g (103, 25 %)

Die Aufreinigung des Rohproduktes erfolgt über Flash-Chromatographie (stationäre Phase: Kieselgel 60, Korngröße: 15 - 40 µm, vorgepackt von der Firma Götec-Labortechnik GmbH, mobile Phase: Chloroform, Methanol (mit 0,1% HOAc), Fluss: 60 ml/min, Beladung: ca. 2 g, Gradient: von 100 % auf 75 % Chloroform in 18 min). Die Produktfraktionen (Rt = 9,1 min) werden vereinigt und bis zur Trockene eingeengt.
Auswaage: 3,18 g (77 %)

### 1.4.2 Festphasen-Peptidsynthese

Peptide werden mit einem Synthesizer der Firma Applied Biosystems GmbH (Carlsbad, CA, USA), Modell 433A, unter Verwendung der Fmoc/tBu-Strategie hergestellt. Die reaktiven Seitenketten der Aminosäuren werden wie folgt geschützt: Lys(Boc), Glu(tBu) und Tyr(tBu). Rink-Amid-Harz der Firma Rapp-Polymere GmbH (Beladungsgrad: 0,24 mmol/g) dient als feste Phase. Die entsprechenden Aminosäuren, der Fmoc-Lysin(ε-Liponsäure)-OH-Baustein und HBTU werden in 4-fachem Überschuss eingesetzt. Als Lösungsmittel wird NMP verwendet und zur jeweiligen Fmoc-Abspaltung Piperidin benutzt (20% in NMP).

Das geschützte Peptid wird mit TFA:Thioanisol:Anisol = 90:8:2 (1 ml pro 100 mg) vom Harz abgespalten (1 - 2 h), in MTBE ausgefällt, zentrifugiert und getrocknet.

### 1.4.3 Radioaktive lodierung von Peptiden

Die Tyrosin-haltigen Peptide werden mit ¹²⁵Iod mittels der Chloramin-T-Methode markiert. Zur Markierung wirde eine 1 mM Stammlösung in Wasser verwendet. Wenn nötig, wird DMSO zur besseren Löslichkeit zugesetzt. Dazu wird 10 µl der Stammlösung mit 20 µl Phosphatpuffer (0,25 M, pH 7,4) versetzt und die gewünschte Menge an radioaktivem lod zugegeben. Zur Markierung werden 5 µl Chloramin-T (2 mg/ml H₂O) hinzugefügt. Die Reaktion erfolgt für 30 Sekunden und wird anschließend mit 10 µl einer gesättigten Methionin-Lösung abgebrochen.

Nach der Markierung wird das Peptid mittels semi-präparativer HPLC aufgereinigt, um das überschüssige freie Iod und andere Nebenprodukte zu entfernen. 100 µl der 0,1 mM Stammlösung werden jeweils für die Injektion verwendet. Vor der Injektion wird die Radioaktivität durch einen Geigerzähler erfasst.

Auf analoge Weise lassen sich auch Konjugate mit anderen Wirkstoffen herstellen.

### 2. Anwendungsbeispiele

### 2.1. Organverteilungsstudien

Zur Ermittlung der Pharmakokinetik werden die zu untersuchenden radioaktiv markierten Moleküle aus Beispiel 1.1 weiblichen NMRI Mäusen über die Schwanzvene injiziert (etwa 100 µl pro Tier). Anschließend werden die Tiere (n = 3 pro Zeitpunkt) zu den entsprechenden Zeitpunkten getötet, seziert und die Verteilung der Radioaktivität in den isolierten Organen (Leber, Niere, Lunge, Milz, Darm, Gehirn, Herz, Blut, ...) mittels γ-Counter (Berthold LB951G) quantifiziert. Die gemessene Radioaktivität pro Gramm Organ/Gewebe bezogen auf die injizierte Dosis (ID) wird bestimmt und als %ID/g angegeben.

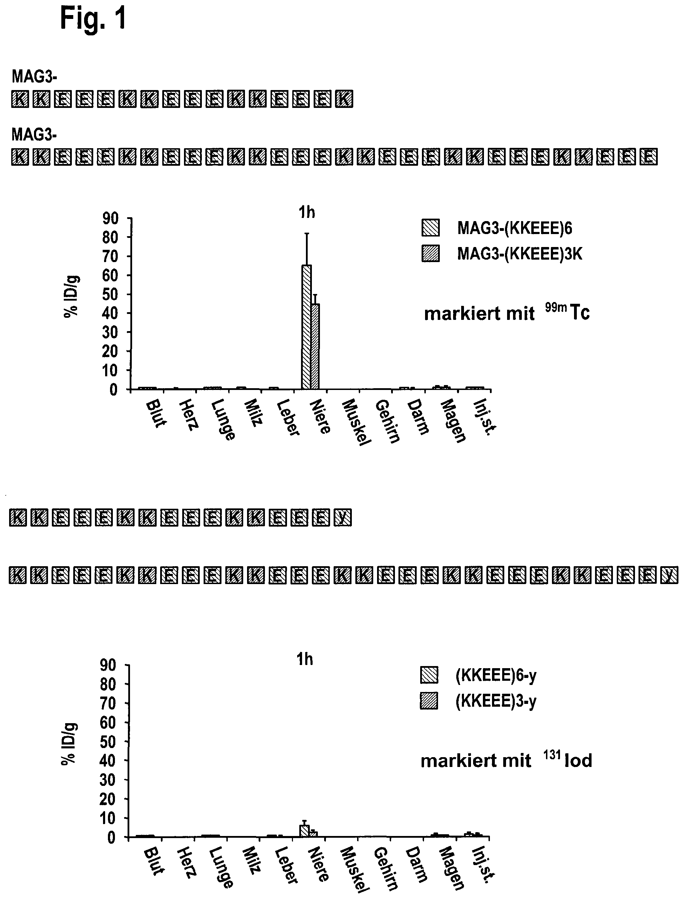

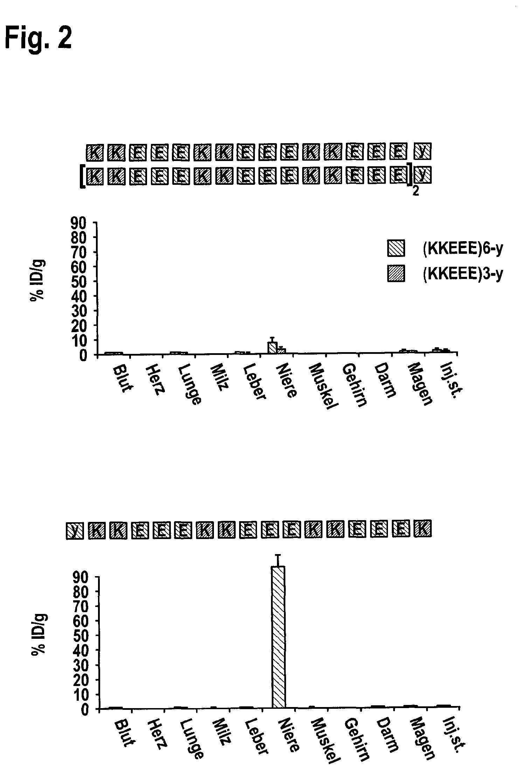

Es wird der Einfluss der Kettenlänge auf die Wirkstofffreisetzung untersucht. Untersucht werden die Strukturen MAG3-KKEEEKKEEEKKEEEK, MAG3-KKEEEKKEEEKKEEEKKEEEKKEEEKKEEE und y-KKEEEKKEEEKKEEEK (N-terminale Verknüpfung des Wirkstoffs - Abbildung 1, oben und Abbildung 2 unten) und die Strukturen KKEEEKKEEEKKEEE-y und KKEEEKKEEEKKEEEKKEEEKKEEEKKEEE-y (C-terminale Verknüpfung des Wirkstoffs - Abbildung 1, unten und Abbildung 2, oben) y steht hierbei für Tyrosin; MAG3 für ein Peptidfragment, welches ^{99m}Tc komplexiert.

Das Ergebnis ist in Abbildung 1 und 2 dargestellt (ID/g steht hierbei für "Injected Dose per Gram of Tissue): Die Freisetzung von radiomarkiertem Tyrosin (als "Wirkstoff") wird zum einen über die Kettenlänge und zum anderen über die Verknüpfungsstelle des "Wirkstoffes" (C- oder N-Terminus) stark beeinflusst. Grundsätzlich führen längere Peptide zu einer verzögerten Freisetzung. Außerdem verläuft die Freisetzung C-terminal-verknüpfter Tracer (lod-Tyrosin oder auch MAG3 mit ^{99m}Tc) wesentlich schneller als bei N-terminaler Verknüpfung. Der geschwindigkeitsbestimmende Schritt beim Peptidabbau wird offensichtlich vor allem durch Carboxypeptidasen beeinflusst, die vom C-Terminus aus beginnend das Peptid abbauen.

Durch den molekularen Aufbau des Peptides und den Ort der Verknüpfung des Wirkstoffes (C- oder N-Terminus) kann die Freisetzungskinetik eines Wirkstoffes bewusst eingestellt werden.

### 2.2. Administrationsroute

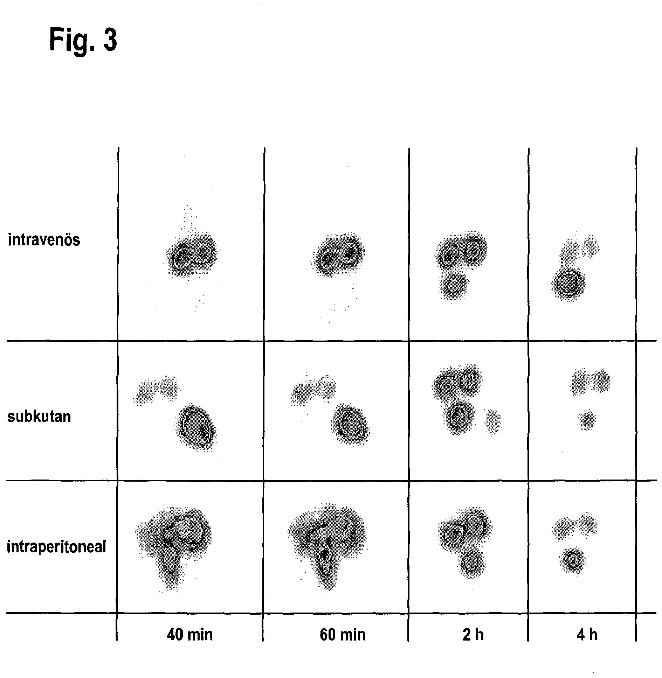

In weiteren Versuchen wird die Administrationsroute untersucht. Dazu werden neun NMRI-Mäuse in drei Gruppen eingeteilt. Alle Tiere erhalten 10 mg/kg KG eines Konjugates von D-Tyrosin N-terminal an (KKEEE)₃K gebunden. Ein Teil des Konjugates wird mittels Chloramin-T-Methode am D-Tyrosin mit 131Iod markiert. Gruppe 1 wird das markierte Konjugat intravenös, Gruppe 2 subkutan und Gruppe 3 intraperitoneal verabreicht. Das Konjugat ist dabei in 100 µL PBS-Puffer gelöst. Zu verschiedenen Zeiten (40, 60, 120 und 240 Minuten) werden nun SPECT-Aufnahmen von Tieren aus der jeweiligen Gruppe erstellt. Die Ergebnisse dieser Versuchsreihe sind in Abbildung 3 dargestellt. Neben der in der Literatur beschriebenen intravenösen Gabe von Peptiden/Proteinen zum Wirkstofftransport in die Nieren kann auch die subkutane und intraperitoneale Gabe der erfindungsgemäßen Peptide bzw. Peptid-Wirkstoff-Konjugate erfolgreich die Nieren adressieren.

### 2.3. Szintigraphische Verteilung von Diacetylkaffeesäure-Konjugaten

In weiteren Versuchen wird der potentielle Wirkstoff Diacetylkaffeesäure (DCA) sowohl N-terminal als auch mehrfach an Lysinseitenketten des Peptidrückgrats angebunden. Die Herstellung des N-terminalen Konjugats mit y(KKEEE)₃K erfolgt analog wie unter 1.2. beschrieben; die Herstellung des zweifach konjugierten Moleküls (Struktur: yKKK(DCA)EEEKKEEEKKK(DCA)EEEK) wie unter 1.3. beschrieben. Die so erhaltenen Peptid-Wirkstoff-Konjugate werden nach der Markierung mittels Iod-125 und intravenöser Applikation im Tiermodell Maus auf ihre Nierenselektivität untersucht.

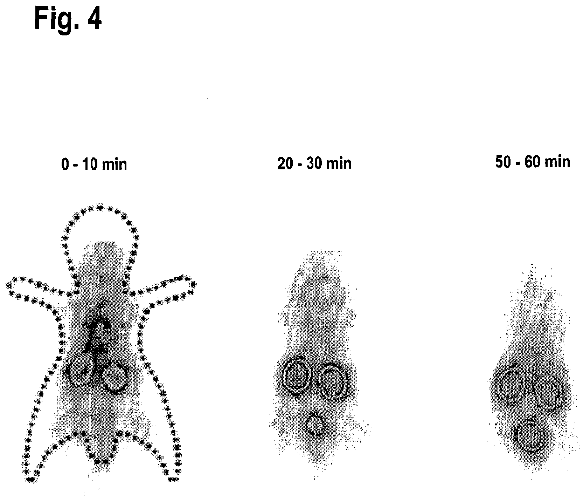

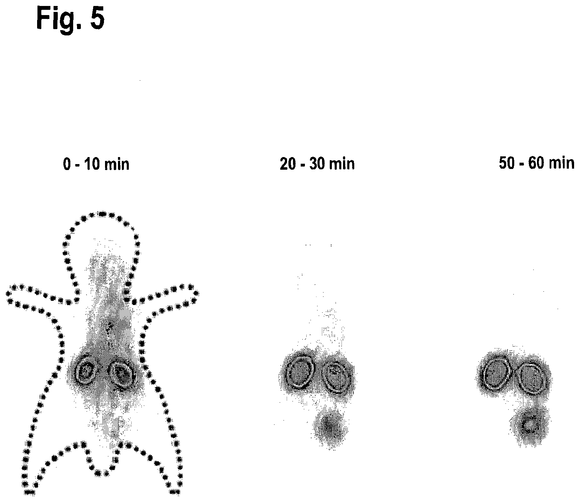

Ergebnis (siehe Abbildung 4 und 5): Die hergestellten Peptid-Wirkstoffkonjugate behalten sowohl nach der N-terminalen Anbindung von Diacetylkaffeesäure (Abbildung 4) als auch bei 2facher Anbindung von Diacetylkaffeesäure an unterschiedliche Seitenketten von Lysin des Peptidrückgrats (Abbildung 5) ihre hohe Nierenspezifität bei.

### 2.4. Schutz der Niere

In einer präklinischen Studie werden BALB/c-Mäuse mit Doxorubicin behandelt. Jedes Versuchstier erhält eine Dosis von 11 mg/kg Körpergewicht. Einer Kontrollgruppe wird lediglich eine isotonische Kochsalzlösung verabreicht. Die mit Doxorubicin behandelten Tiere werden in verschiedene Gruppen aufgeteilt. Gruppe B erhält zusätzlich zu dem injizierten Doxorubicin eine Injektion von freier Liponsäure in Form ihres Kaliumsalzes. Bei Gruppe C wird statt der freien Liponsäure das Liponsäure-Peptid-Konjugat LA-(KKEEE)₃ (Liponsäure *N*-terminal an (Lys-Lys-Glu-Glu-Glu)₃) per Injektion verabreicht.

| | Kontrolle | Doxorubicin | Doxorubicin + Liponsäure-Konjugat | Doxorubicin + Liponsäure |
|---|---|---|---|---|
| Tierzahl | 6 | 6 | 6 pro Dosis; bei 3 Dosen = 18 Tiere | 6 pro Dosis; bei 3 Dosen = 18 Tiere |
| Behandlung | 0,9% Salzlösung | Doxorubicin-Hydrochlorid verdünnt in 0,9% Salzlösung auf 11 mg/kg KG | Doxorubicin-Hydrochlorid verdünnt in 0,9% Salzlösung auf 11 mg/kg KG | Doxorubicin-Hydrochlorid verdünnt in 0,9% Salzlösung auf 11 mg/kg KG |

### 2.5 Szintigraphische Verteilung von Liponsäure-Konjugaten nach Beispiel 1.4

In weiteren Versuchen wird der potentielle Wirkstoff Liponsäure über die Lysinseitenketten des Peptidrückgrats angebunden. Die Herstellung des Konjugats y(KKKε(Liponsäure)EEE)₃K erfolgt wie in Beispiel 1.4. beschrieben. Das so erhaltene Peptid-Wirkstoff-Konjugat wird nach der Markierung mittels lod-125 und intravenöser Applikation im Tiermodell Maus auf seine Nierenselektivität untersucht.

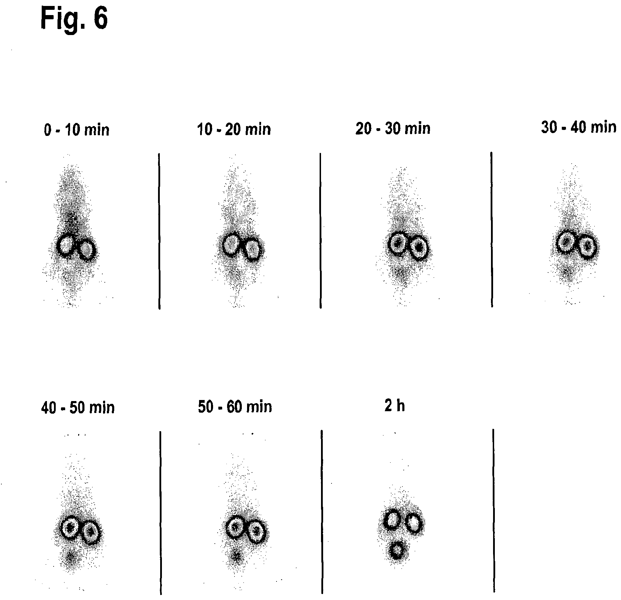

Ergebnis (siehe Abbildung 6): Das hergestellte Peptid-Wirkstoffkonjugat weist eine hohe Nierenspezifität auf.

## Patentansprüche

1. Konjugat enthaltend mindestens ein nierenselektives Trägermolekül und mindestens einen Wirkstoff, der eine schützende Wirkung für die Niere vor nephrotoxischen Wirkstoffen aufweist, **dadurch gekennzeichnet, dass** das mindestens eine nierenselektive Trägermolekül ein Peptid ist, welches zu mehr als 50 % (bezogen auf die Anzahl der Aminosäureeinheiten) aus Sequenzabschnitten ausgewählt aus der Gruppe umfassend -(KKEEE)-, -(RREEE)-, - (KKEE)-, -(KKKEEE)- und -(KKKEE)- besteht
und wobei
- das Peptid zu mindestens 80 % (bezogen auf die Anzahl der Aminosäureeinheiten) aus den Aminosäuren K und E bzw. R und E besteht,
- und das Peptid 3 bis 5 Sequenzabschnitte wie zuvor definiert enthält.

2. Konjugat nach Anspruch 1, **dadurch gekennzeichnet, dass** das Peptid für ein Peptid ausgewählt aus der Gruppe umfassend (RREEE)₃R, (KKEE)₅K, (KKKEE)₃K, (KKKEEE)₃K und (KKEEE)₃K steht.

3. Konjugat nach Anspruch 1 oder 2, **dadurch gekennzeichnet, dass** der mindestens eine Wirkstoff ausgewählt ist aus Antioxidantien, Apoptoseinhibitoren, Wirkstoffen mit Einfluss auf den Zellzyklus, Wirkstoffen die die Reparaturmechanismen der Zellen aktivieren, Rezeptorinhibitoren und Kombinationen davon.

4. Konjugat nach Anspruch 3, **dadurch gekennzeichnet, dass** der mindestens eine Wirkstoff ein Antioxidans und/ oder ein Apoptoseinhibitor ist.

5. Konjugat nach Anspruch 4, **dadurch gekennzeichnet, dass** der Wirkstoff ausgewählt ist aus Liponsäure, Resveratrol, Kaffeesäure, Luteolin, Quercetin, Rutin, Cyanidin, Xanthohumol, Ascorbinsäure, Nicotinsäure, Amifostin, Alliin, Thiole, Tocopherole, Carotinoide, Butylhydroxytoluol (BHT), Pifithrin-µ, Pifithrin-α, MDL 28170 und/ oder NS3694.

6. Verfahren zur Herstellung eines Konjugats nach einem oder mehreren der Ansprüche 1 bis 5, **dadurch gekennzeichnet, dass** ein optional aktivierter Wirkstoff, der eine schützende Wirkung auf die Niere vor nephrotoxischen Wirkstoffen aufweist, an das Trägermolekül konjugiert wird.

7. Konjugat nach einem oder mehreren der Ansprüche 1 bis 5 zur Verwendung als Arzneimittel.

8. Konjugat nach einem oder mehreren der Ansprüche 1 bis 5 zur Verwendung zum Schutz der Niere vor nephrotoxischen Wirkstoffen.

9. Arzneimittel enthaltend mindestens ein Konjugat nach einem oder mehreren der Ansprüche 1 bis 5.

## Claims

1. Conjugate containing at least one kidney-selective carrier molecule and at least one active compound which has a protective action for the kidney against nephrotoxic active compounds, **characterised in that** the at least one kidney-selective carrier molecule is a peptide which consists of more than 50% (based on the number of amino acid units) of sequence sections selected from the group comprising -(KKEEE)-, -(RREEE)-, -(KKEE)-, -(KKKEEE)- and -(KKKEE)-
and where
- the peptide consists of at least 80% (based on the number of amino acid units) of amino acids K and E or R and E,
- and the peptide contains 3 to 5 sequence sections as defined above.

2. Conjugate according to Claim 1, **characterised in that** the peptide stands for a peptide selected from the group comprising (RREEE)₃R, (KKEE)₅K, (KKKEE)₃K, (KKKEEE)₃K and (KKEEE)₃K.

3. Conjugate according to Claim 1 or 2, **characterised in that** the at least one active compound is selected from antioxidants, apoptosis inhibitors, active compounds having an influence on the cell cycle, active compounds which activate the repair mechanisms of the cells, receptor inhibitors and combinations thereof.

4. Conjugate according to Claim 3, **characterised in that** the at least one active compound is an antioxidant and/or an apoptosis inhibitor.

5. Conjugate according to Claim 4, **characterised in that** the active compound is selected from lipoic acid, resveratrol, caffeic acid, luteolin, quercetin, rutin, cyanidin, xanthohumol, ascorbic acid, nicotinic acid, amifostin, alliin, thiols, tocopherols, carotinoids, butylhydroxytoluene (BHT), pifithrin-µ, pifithrin-α, MDL 28170 and/ or NS3694.

6. Process for the preparation of a conjugate according to one or more of Claims 1 to 5, **characterised in that** an optionally activated active compound which has a protective action on the kidney against nephrotoxic active compounds is conjugated onto the carrier molecule.

7. Conjugate according to one or more of Claims 1 to 5 for use as medicament.

8. Conjugate according to one or more of Claims 1 to 5 for use for protection of the kidney against nephrotoxic active compounds.

9. Medicament comprising at least one conjugate according to one or more of Claims 1 to 5.

## Revendications

1. Conjugué contenant au moins une molécule véhicule sélective du rein et au moins un composé actif qui possède une action protectrice vis-à-vis du rein contre des composés actifs néphrotoxiques, **caractérisé en ce que** la au moins une molécule véhicule sélective du rein est un peptide qui est constitué à plus de 50% (sur la base du nombre d'unités d'acides aminés) de sections de séquences choisies dans le groupe constitué par -(KKEEE)-, -(RREEE)-, -(KKEE)-, -(KKKEEE)- et -(KKKEE)-
et où
- le peptide est constitué d'au moins 80% (sur la base du nombre d'unités d'acides aminés) des acides aminés K et E ou R et E,
- et le peptide contient de 3 à 5 sections de séquences telles que définies ci-dessus.

2. Conjugué selon la revendication 1, **caractérisé en ce que** le peptide représente un peptide choisi dans le groupe constitué par (RREEE)₃R, (KKEE)₅K, (KKKEE)₃K, (KKKEEE)₃K et (KKEEE)₃K.

3. Conjugué selon la revendication 1 ou 2, **caractérisé en ce que** le au moins un composé actif est choisi parmi les antioxydants, les inhibiteurs d'apoptose, les composés actifs ayant une influence sur le cycle cellulaire, les composés actifs qui activent les mécanismes de réparation des cellules, les inhibiteurs de récepteurs et des combinaisons de ceux-ci.

4. Conjugué selon la revendication 3, **caractérisé en ce que** le au moins un composé actif est un antioxydant et/ou un inhibiteur d'apoptose.

5. Conjugué selon la revendication 4, **caractérisé en ce que** le composé actif est choisi parmi l'acide lipoïque, le resvératrol, l'acide caféique, la lutéoline, la quercétine, la rutine, la cyanidine, le xanthohumol, l'acide ascorbique, l'acide nicotinique, l'amifostine, l'alliine, les thiols, les tocophérols, les caroténoïdes, le butylhydroxytoluène (BHT), la pifithrine-µ, la pifithrine-a, MDL 28170 et/ou NS3694.

6. Procédé de préparation d'un conjugué selon l'une ou plusieurs parmi les revendications 1 à 5, **caractérisé en ce qu'**un composé actif éventuellement activé qui possède une action protectrice vis-à-vis du rein contre des composés actifs néphrotoxiques est conjugué sur la molécule véhicule.

7. Conjugué selon l'une ou plusieurs parmi les revendications 1 à 5, pour une utilisation comme médicament.

8. Conjugué selon l'une ou plusieurs parmi les revendications 1 à 5, pour une utilisation pour la protection du rein contre des composés actifs néphrotoxiques.

9. Médicament comprenant au moins un conjugué selon l'une ou plusieurs parmi les revendications 1 à 5.
